# FASCICULE DE BREVET EUROPEEN

(11) **EP 4 649 304 B1**
(45) Date de publication et mention de la délivrance du brevet: **21.10.2026**
(21) Numéro de dépôt: 24703594.2
(22) Date de dépôt: 11.01.2024
(51) Int. Cl.: G01N 27/02, A61B 5/0536

(54) **MOTIF DE MESURE POUR UN SYSTEME ET UN PROCÉDÉ DE TOMOGRAPHIE PAR PROCESSUS ELECTRIQUE**
MESSMUSTER FÜR EIN SYSTEM UND EIN VERFAHREN ZUR ELEKTROPROZESSTOMOGRAPHIE
MEASUREMENT PATTERN FOR AN ELECTRICAL PROCESS TOMOGRAPHY SYSTEM AND METHOD

(30) Priorité: 13.01.2023 FR 2300362; 21.11.2023 FR 2312833
(43) Date de publication de la demande: 19.11.2025
(73) Titulaire: Touch Sensity, 33600 Pessac (FR)
(72) Inventeur: BRIANT, Marc, 33600 Pessac (FR)
(74) Mandataire: Plasseraud IP
(86) Numéro de dépôt international: PCT/FR2024/050034
(87) Numéro de publication internationale: WO 2024/149959

(56) Documents cités:
- US-A1- 2018 169 794
- ALLER MARTÍN ET AL: "Study and comparison of different Machine Learning-based approaches to solve the inverse problem in Electrical Impedance Tomographies", vol. 35, no. 7, 4 November 2022 (2022-11-04), London, pages 5465 - 5477, XP093067625, ISSN: 0941-0643, Retrieved from the Internet <URL:https://link.springer.com/article/10.1007/s00521-022-07988-7/fulltext.html> [retrieved on 20230726], DOI: 10.1007/s00521-022-07988-7
- TARABI NAZILLA ET AL: "Experimental evaluation of some current injection-voltage reading patterns in electrical impedance tomography (EIT) and comparison to simulation results - Case study: Large scales", FLOW MEASUREMENT AND INSTRUMENTATION, BUTTERWORTH-HEINEMANN, OXFORD, GB, vol. 83, 23 November 2021 (2021-11-23), XP086936112, ISSN: 0955-5986, [retrieved on 20211123], DOI: 10.1016/J.FLOWMEASINST.2021.102087

## Description

Le domaine de l'invention se rapporte à la tomographie par processus électrique (aussi connue sous l'acronyme anglophone EPT pour « electrical process tomography ») d'un substrat.

La tomographie par processus électrique est une technique d'imagerie qui consiste à déterminer les propriétés électriques d'un substrat à partir de mesures de tension de paires d'électrodes disposées à la périphérie du substrat. Les mesures de tension sont utilisées pour cartographier la densité de charge électrique ou la conductivité électrique du substrat.

La tomographie par processus électrique couvre différents procédés, notamment la tomographie par impédance électrique (aussi connue sous l'acronyme anglophone EIT pour « electrical impedance tomography ») et la tomographie par résistance électrique (aussi connue sous l'acronyme anglophone ERT pour « electrical resistance tomography »). La tomographie par résistance électrique est particulièrement appropriée lorsque la partie réelle de l'impédance du substrat prédomine sur la partie complexe.

La tomographie par impédance électrique (TIE) présente des applications dans le domaine médical. À titre d'exemple, la demande internationale WO 2020/094628 A1 décrit un textile intelligent formé d'un tissu conducteur sur lequel sont placées des électrodes pour détecter des mouvements articulaires dans le cadre de la prévention de troubles musculosquelettiques.

Il est également connu d'utiliser la TIE dans un contexte industriel pour analyser un substrat liquide, par exemple un fluide circulant dans une canalisation. Par ailleurs, la TIE peut être appliquée à un matériau solide pour en connaître la structure et visualiser la manière dont les différentes zones du matériau conduisent ou s'opposent au passage du courant afin d'y détecter des anomalies telles que des fissures.

Le principe général de la TIE est de disposer des électrodes à la périphérie du substrat à analyser, d'alimenter des doublets d'électrodes avec une source de courant ou de tension et de mesurer la tension entre les deux électrodes de différentes paires d'électrodes. Plusieurs processus ont ainsi été développés sur ce principe et se différencient par leur motif d'injection - ou « injection pattern » dans la littérature anglophone -, c'est-à-dire la manière dont sont formés les doublets d'électrodes à stimuler.

Le motif d'injection le plus utilisé est le motif adjacent - ou « adjacent pattern » - décrit par D.C. Barber et B.H. Brown dans l'article « Applied potential tomography » (Journal of Physics E: Scientific Instruments, vol. 17, n°9, 1984) et qui consiste à alimenter chaque doublet d'électrodes formé de deux électrodes voisines puis à mesurer la tension au niveau de chaque paire d'électrodes formée également de deux électrodes voisines.

Toutefois, un tel motif d'injection fournit peu d'informations sur le centre du substrat puisque la stimulation et la mesure sont réalisées sur le bord de celui-ci.

D'autres processus ont été proposés pour combler cette lacune et sont notamment discutés par M. Haingartner et al. dans l'article « Improved current injection pattern for the detection of delaminations in carbon fiber reinforced polymer plates using electrical impedance tomography » (Structural Health Monitoring, vol. 20, n°5, 2021, p. 2747-2757). Le substrat à analyser est une plaque en polymère renforcé de fibres de carbone (plus connu sous l'acronyme anglophone CFRP pour « carbon filter reinforced polymer ») dont la forme rectangulaire se prête à des motifs d'injection variés. Cette publication rappelle que, outre le motif adjacent, il est possible d'utiliser un motif opposé - ou « opposite pattern » - dans lequel chaque doublet d'électrodes est formé de deux électrodes symétriques par rapport à un axe vertical ou horizontal ou par rapport au centre de la plaque. Un motif diagonal - ou « diagonal pattern » -, dans lequel chaque doublet d'électrodes est formé de deux électrodes symétriques par rapport à une diagonale du substrat, est aussi mentionné. De même que le motif adjacent, il est prévu dans ces deux motifs d'injection de mesurer la tension de paires d'électrodes voisines.

Le motif opposé et le motif diagonal permettent de connaître les propriétés électriques du centre du matériau. Néanmoins, ces motifs d'injection restent sensibles aux conditions de bord et sont donc peu adaptés aux matériaux hétérogènes tels que des matériaux composites. De plus, les stimulations successives de doublets d'électrodes éloignées sur le matériau créent des directions de propagation du courant opposées qui sont susceptibles de se compenser et donc d'altérer les mesures de tension.

Des motifs d'injection plus complets ont été développés. B. Loyola et al. ont proposé, dans l'article « Detection of spatially distributed damage in fiber-reinforced polymer composites » (Structural Health Monitoring, vol. 12, n°3, 2013, p. 225-239), de combiner le motif opposé et le motif diagonal pour des matériaux anisotropes. Par ailleurs, l'article de M. Haingartner et al. (2021) cité précédemment décrit un motif en étoile - ou « star pattern » - qui consiste à stimuler tous les doublets possibles d'électrodes, excepté les électrodes voisines sur un même côté de la plaque.

Ces motifs d'injection présentent le désavantage d'avoir un coût de temps élevé et de nécessiter des ressources de calcul conséquentes.

Une autre solution possible pour obtenir des informations sur le centre du substrat consiste à adapter le motif de mesure plutôt que le motif d'injection. À ce titre, M. Aller et al. décrivent, dans l'article « Study and comparison of different Machine Learning-based approaches to solve the inverse problem in Electrical Impedance Tomographies » (Neural Computing and Applications, vol. 35, n°7, 2022, p. 5465-5477), un motif de mesure opposé - qualifié là aussi de « opposite pattern » - dans lequel chaque paire d'électrodes est formée de deux électrodes symétriques par rapport au centre d'un substrat présentant un contour circulaire. Le motif d'injection utilisé est le motif adjacent proposé par D.C. Barber et B.H. Brown (1984).

La présente invention vient améliorer la situation.

A ce titre, l'invention concerne un système de tomographie selon la revendication 1 par processus électrique, comprenant un dispositif et un substrat.

Le dispositif comprend :
- une pluralité d'électrodes reliée à une portion du substrat,
- une source d'alimentation électrique agencée pour être connectée à un doublet d'électrodes de la pluralité d'électrodes,
- un capteur de tension agencé pour être connecté à une paire d'électrodes de la pluralité d'électrodes,
- une unité de commande agencée pour commander l'alimentation successive par la source d'alimentation électrique de doublets d'électrodes formés chacun de deux électrodes voisines, et pour commander, à chaque fois qu'un doublet d'électrodes est alimenté, des mesures de tension respectives par le capteur de tension, chacune entre deux électrodes de paires d'électrodes associées selon un motif de mesure, et
- un calculateur agencé pour cartographier, à partir des mesures de tension, la densité de charge électrique ou la conductivité électrique de la portion du substrat.

Une section du substrat, définie par l'intersection d'un plan avec la portion du substrat, est divisée en secteurs angulaires par un ensemble de droites sécantes en un même point de la section du substrat, deux secteurs angulaires délimités par les deux mêmes droites formant des secteurs angulaires opposés.

La pluralité d'électrodes est reliée à la portion du substrat de sorte que, par projection sur la section du substrat, deux secteurs angulaires opposés sont tous les deux dépourvus d'électrodes ou tous les deux pourvus d'au moins deux électrodes.

Le motif de mesure respecte les deux conditions suivantes :
- chaque paire d'électrodes du motif de mesure est formée de deux électrodes de secteurs angulaires opposés, et
- deux paires d'électrodes du motif de mesure associées à de mêmes secteurs angulaires sont telles que les deux segments reliant chacun, par projection sur la section du substrat, les deux électrodes respectives des deux paires d'électrodes du motif de mesure ne sont pas sécants.

Dans un ou plusieurs modes de réalisation, la pluralité d'électrodes est reliée par projection sensiblement à la périphérie de la section du substrat.

Dans un ou plusieurs modes de réalisation, la pluralité d'électrodes est reliée par projection à une ligne fermée dotée d'un sens de parcours de la section du substrat et deux électrodes voisines sont deux électrodes consécutives de la ligne fermée suivant le sens de parcours.

Dans un ou plusieurs modes de réalisation, le calculateur est agencé pour cartographier la densité de charge électrique ou la conductivité électrique de la portion du substrat par la résolution d'un problème inverse dont des entrées sont les mesures de tension.

Dans un ou plusieurs modes de réalisation, la source d'alimentation électrique est une source de tension ou une source de courant.

L'invention concerne également un procédé de tomographie selon la revendication 6 par processus électrique d'un substrat, comprenant les opérations suivantes :
- relier une pluralité d'électrodes à une portion du substrat,
- alimenter successivement par une source d'alimentation électrique des doublets d'électrodes formés chacun de deux électrodes voisines,
- pour chaque alimentation d'un doublet d'électrodes, effectuer des mesures de tension respectives par un capteur de tension, chacune entre deux électrodes de paires d'électrodes associées selon un motif de mesure, et
- cartographier par un calculateur, à partir des mesures de tension, la densité de charge électrique ou la conductivité électrique de la portion du substrat.

Une section du substrat, définie par l'intersection d'un plan avec la portion du substrat, est divisée en secteurs angulaires par un ensemble de droites sécantes en un même point de la section du substrat, deux secteurs angulaires délimités par les deux mêmes droites formant des secteurs angulaires opposés.

La pluralité d'électrodes est reliée à la portion du substrat de sorte que, par projection sur la section du substrat, deux secteurs angulaires opposés sont tous les deux dépourvus d'électrodes ou tous les deux pourvus d'au moins deux électrodes.

Le motif de mesure respecte les deux conditions suivantes :
- chaque paire d'électrodes du motif de mesure est formée de deux électrodes de secteurs angulaires opposés, et
- deux paires d'électrodes du motif de mesure associées à de mêmes secteurs angulaires sont telles que les deux segments reliant chacun, par projection sur la section du substrat, les deux électrodes respectives des deux paires d'électrodes du motif de mesure ne sont pas sécants.

Dans un ou plusieurs modes de réalisation, la source d'alimentation électrique alimente tous les doublets possibles d'électrodes voisines.

Avantageusement, la pluralité d'électrodes est reliée à la portion du substrat de sorte que, par projection sur la section du substrat, deux secteurs angulaires opposés sont pourvus du même nombre d'électrodes. Pour chaque alimentation d'un doublet d'électrodes, des secteurs angulaires opposés sont munis d'une bijection entre leurs électrodes respectives, et le motif de mesure est tel que chaque paire d'électrodes est formée de deux électrodes respectivement antécédent et image par la bijection associée aux secteurs angulaires opposés correspondants.

La bijection associée à deux secteurs angulaires opposés est par exemple invariante de l'alimentation d'un doublet d'électrodes à une autre.

Dans un ou plusieurs modes de réalisation, le capteur de tension effectue des mesures de tension respectives, chacune entre les deux électrodes de toutes les paires possibles d'électrodes associées selon le motif de mesure.

D'autres caractéristiques, détails et avantages apparaîtront à la lecture de la description détaillée ci-après, et à l'analyse des dessins annexés sur lesquels :
[Fig. 1] illustre un système de tomographie par processus électrique selon l'invention ;
[Fig. 2] illustre un procédé de tomographie par processus électrique d'un substrat selon l'invention ;
[Fig. 3] illustre une opération de positionnement des électrodes sur le substrat du procédé de la [Fig. 2] ; et
[Fig. 4] illustre une variante du motif de mesure de l'invention.

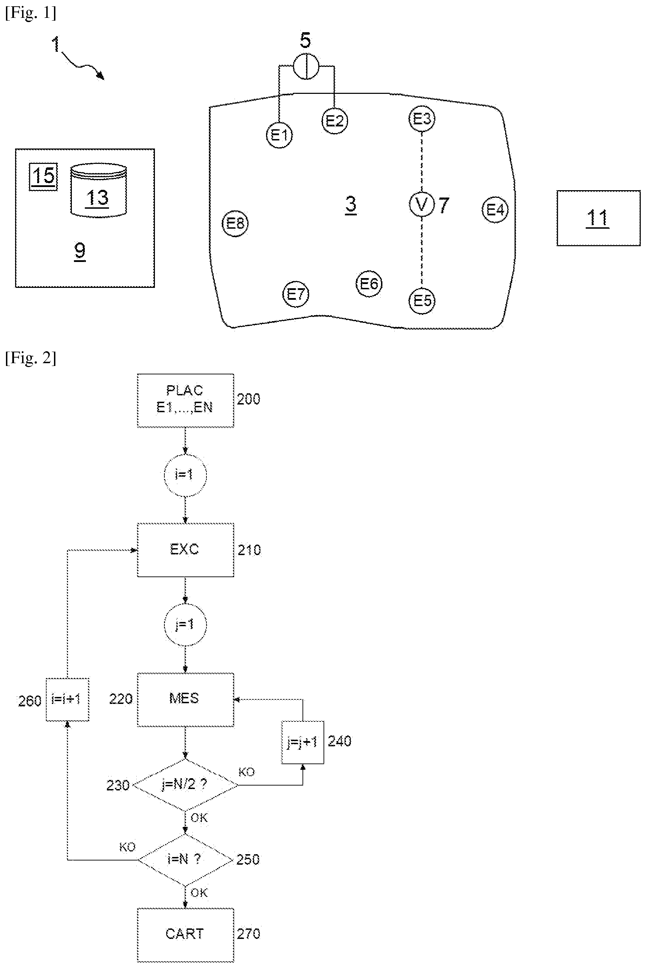

La [Fig. 1] illustre un système de tomographie par processus électrique comprenant un dispositif 1 et un substrat 3.

Le dispositif 1 est agencé pour appliquer une tomographie par processus électrique au substrat 3 afin d'en déterminer les propriétés électriques. Dans la suite de la description, on considère, sans perte de généralité, que le dispositif 1 est agencé pour mettre en œuvre une tomographie par impédance électrique, désignée ci-après par l'acronyme TIE. Toutefois, le dispositif 1 peut également être adapté pour mettre en œuvre une tomographie par résistance électrique.

Le substrat 3 peut être formé de tout type de matériau, liquide ou solide, homogène ou hétérogène.

Par exemple, le substrat 3 est un fluide circulant dans une canalisation ou un liquide stocké dans une cuve industrielle.

Le substrat 3 peut également être un matériau composite, c'est-à-dire un matériau formé d'une matrice et d'un renfort. La matrice peut être organique, en plastique, en métal, en céramique, en graphite ou en être une combinaison. Dans un composite à matrice organique, la matrice - alors appelée résine - est par exemple thermodurcissable : résine époxy, polyester ou encore polyimide. La matrice organique peut aussi être thermoplastique : polypropylène (PP), polyamide (PA) ou encore polyétheréthercétone (PEEK). Le renfort peut comprendre des fibres de carbone, des fibres de verre, des fibres d'aramide ou des fibres végétales. Le renfort peut également comprendre des charges, par exemple des charges métalliques, des charges de carbone, des charges de silice ou encore des charges de polymère.

Typiquement, le substrat 3 est un composite carbone/carbone - ou C/C - formé d'une matrice de graphite renforcée de fibres de carbone ou un polymère renforcé de fibres de carbone - ou CFRP pour « carbon filter reinforced polymer ».

La TIE permet d'obtenir une distribution de la densité de charge électrique ou de la conductivité électrique d'une portion du substrat 3. Dans le cas particulier où le substrat 3 est un matériau multicouche, la portion du substrat 3 peut être une couche du matériau.

La TIE peut être utilisée pour détecter des anomalies - généralement des fissures, mais aussi un impact ou un délaminage - qui modifient la manière dont le substrat 3 conduit le courant.

Comme illustré sur la [Fig. 1], le dispositif 1 comprend une pluralité d'électrodes, une source 5 d'alimentation électrique, un capteur 7 de tension, une unité de commande 9 et un calculateur 11.

Les électrodes de la pluralité d'électrodes sont agencées pour être reliées à une portion du substrat 3 dont on souhaite connaître les propriétés électriques.

Lorsque le substrat 3 est liquide, les électrodes sont positionnées sur le contenant, par exemple une canalisation, une cuve ou un réservoir.

Lorsque le substrat 3 est solide, les électrodes sont par exemple directement déposées sur sa surface extérieure. Dans un matériau multicouche, les électrodes peuvent être déposées sur une des couches du matériau ou encore être en contact avec plusieurs - voire la totalité - des couches. Les électrodes peuvent notamment percer le matériau pour être en contact avec une ou plusieurs couches.

Les positions des électrodes délimitent la portion - bidimensionnelle ou tridimensionnelle - dont on souhaite déterminer la distribution de la densité de charge électrique ou de la conductivité électrique. Par ailleurs, le positionnement des électrodes peut être caractérisé par une projection - typiquement une projection orthogonale - sur l'intersection d'un plan avec la portion du substrat 3. Une telle intersection est appelée « section » dans la suite de la description. On peut considérer que les électrodes sont reliées par projection à la section du substrat 3.

La pluralité d'électrodes peut comprendre un nombre pair d'électrodes. Par exemple, dans le mode de réalisation représenté sur la [Fig. 1], la pluralité d'électrodes comprend huit électrodes respectivement référencées E1, E2, E3, E4, E5, E6, E7 et E8. Bien évidemment, la pluralité d'électrodes peut comprendre plus ou moins de huit électrodes. À titre d'exemple, il est connu d'avoir recours à seize électrodes, comme proposé, entre autres, par G. Pugach et al. dans l'article « Neural learning of the topographic tactile sensory information of an artificial skin through a self-organizing map » (Advanced Robotics, vol. 29, n°21, 2015, p. 1393-1409), pour mettre en œuvre une TIE. Par ailleurs, la pluralité d'électrodes peut également comprendre un nombre impair d'électrodes.

La source 5 est agencée pour être connectée à un doublet d'électrodes de la pluralité d'électrodes. Plus particulièrement, la source 5 est agencée pour alimenter successivement des doublets d'électrodes associées selon un motif d'injection prédéterminé. Dans le cadre de la TIE, la stimulation de doublets d'électrodes a pour but de faire circuler le courant au sein du substrat 3 pour observer la manière dont les différentes zones de la portion du substrat 3 conduisent ou s'opposent au passage du courant.

Dans le contexte de l'invention, le motif d'injection utilisé est celui proposé par D.C. Barber et B.H. Brown (1984) : chaque doublet d'électrodes est formé de deux électrodes voisines. Le motif adjacent présente l'avantage, contrairement au motif opposé et au motif diagonal, de ne pas créer de directions de propagation du courant opposées qui sont susceptibles de se compenser et donc d'altérer les mesures de tension ultérieures.

Dans l'exemple de la [Fig. 1], la source 5 est ainsi connectée au doublet d'électrodes voisines E1 et E2.

La source 5 peut être une source de tension, auquel cas la source 5 applique une tension entre les deux électrodes du doublet d'électrodes. En variante, la source 5 est une source de courant, auquel cas la source 5 génère un courant qui circule d'une électrode à l'autre du doublet d'électrodes.

Le capteur 7 est agencé pour être connecté à une paire d'électrodes de la pluralité d'électrodes. Plus particulièrement, le capteur 7 est agencé pour mesurer la tension respectivement entre deux électrodes de plusieurs paires d'électrodes, les deux électrodes de chaque paire d'électrodes étant associées selon un motif de mesure prédéterminé. Dans le cadre de la TIE, les mesures de tension, réalisées pour chaque alimentation d'un doublet d'électrodes, sont destinées à être exploitées par le calculateur 11 pour cartographier la densité de charge électrique ou la conductivité électrique de la portion du substrat 3 à laquelle est reliée la pluralité d'électrodes.

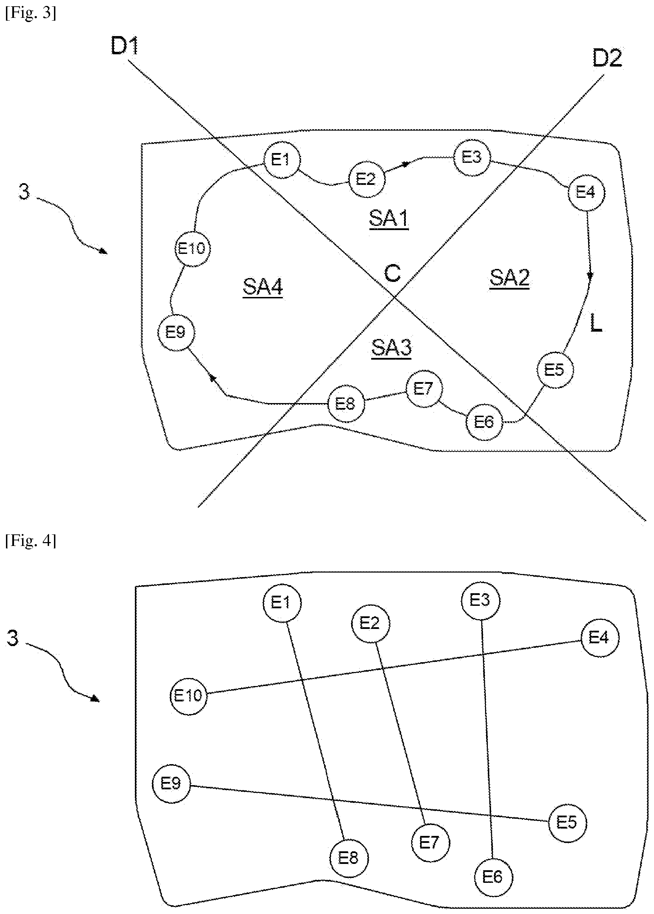

Les détails du motif de mesure faisant l'objet de la présente invention seront détaillés dans la suite de la description, notamment en référence à la [Fig. 2] et à la [Fig. 3].

Dans l'exemple illustré sur la [Fig. 1], le capteur 7 est connecté à la paire d'électrodes E3 et E5.

Le capteur 7 est par exemple un instrument de mesure de type voltmètre.

L'unité de commande 9 est agencée pour commander à la fois la source 5 et le capteur 7. Plus particulièrement, l'unité de commande 9 est agencée pour commander l'alimentation successive, par la source 5, de doublets d'électrodes voisines et pour commander, à chaque fois qu'un doublet d'électrodes est alimenté, des mesures de tension. Chaque mesure de tension est effectuée entre deux électrodes associées selon le motif de mesure et formant ensemble une paire d'électrodes.

L'unité de commande 9 comprend une mémoire 13 et un processeur 15.

La mémoire 13 est agencée pour stocker des instructions dont la mise en œuvre, par le processeur 15, se traduit par le fonctionnement de l'unité de commande 9.

La mémoire 13 peut également stocker les informations et mesures nécessaires au calculateur 11 pour cartographier la densité de charge électrique ou la conductivité électrique de la portion du substrat 3. Par exemple, la mémoire 13 peut conserver un registre dans lequel sont mises en correspondance, d'une part, des données d'identification des doublets d'électrodes et, d'autre part, les mesures de tension.

Les données d'identification d'un doublet d'électrodes comprennent les positions respectives des deux électrodes du doublet d'électrodes dans un repère du substrat 3 ainsi que la valeur de la tension ou de l'intensité du courant appliquée par la source 5 au doublet d'électrodes. Les mesures de tension associées à un doublet d'électrodes correspondent aux mesures réalisées par le capteur 7 entre deux électrodes de plusieurs paires d'électrodes. Chaque mesure de tension est assortie des positions respectives des deux électrodes de la paire d'électrodes correspondante dans le repère du substrat 3.

Bien entendu, il est également possible que les mesures ne soient connues que du calculateur 11 et ne transitent donc pas par l'unité de commande 9. Par ailleurs, les positions des deux électrodes de la paire d'électrodes à laquelle correspond chaque mesure de tension peuvent se déduire de l'ordre dans lequel les mesures de tensions sont réalisées.

La mémoire 13 peut désigner tout support de stockage de données agencé pour recevoir et conserver des données numériques, par exemple un disque dur, un disque à semi-conducteurs (plus connu sous l'acronyme anglophone SSD pour « solid-state drive ») ou plus généralement tout matériel informatique permettant le stockage de données sur de la mémoire flash. La mémoire 13 peut également être une mémoire vive ou encore un disque magnéto-optique. Une combinaison de plusieurs supports de stockages peut aussi être envisagée.

Le processeur 15 peut être réalisé de toute manière connue, par exemple sous la forme d'un microprocesseur, d'un circuit logique programmable (plus connu sous l'acronyme anglophone PLD pour « Programmable Logical Device ») ou d'une puce dédiée de type FPGA (acronyme anglophone pour « Field Programmable Gate Array ») ou SoC (acronyme anglophone pour « System on Chip »), d'une grille de ressources informatiques, d'un microcontrôleur ou de toute autre forme propre disposant de la puissance de calcul nécessaire à la mise en œuvre du procédé de tomographie par processus électrique décrit ci-après. Un ou plusieurs de ces éléments peuvent également être réalisés sous la forme de circuits électroniques spécialisés de type ASIC (acronyme anglophone pour « Application-Specific Integrated Circuit »). Une combinaison de processeurs et de circuits électroniques peut aussi être envisagée.

Enfin, le calculateur 11 est agencé pour cartographier, à partir des mesures de tension, la densité de charge électrique ou la conductivité électrique de la portion du substrat 3.

Il est connu de l'homme du métier que la distribution de la densité de charge électrique ou de la conductivité électrique peut être obtenue par la résolution d'un problème inverse dont des entrées sont les mesures de tension.

Pour ce faire, le calculateur 11 peut communiquer avec l'unité de commande 9 pour recevoir les informations et mesures nécessaires. Dans l'exemple de la [Fig. 1], l'unité de commande 9 et le calculateur 11 sont distincts l'un de l'autre. Toutefois, une telle distinction est fonctionnelle et on comprend que le calculateur 11 peut faire partie intégrante de l'unité de commande 9, auquel cas l'unité de commande 9 et le calculateur 11 forment un seul et même appareil.

Un procédé de tomographie par processus électrique d'un substrat - ici le substrat 3 - va à présent être décrit en référence à la [Fig. 2]. Plus particulièrement, le procédé détaillé ci-après est un procédé de TIE. Toutefois, une tomographie par résistance électrique peut être mise en œuvre de la même manière.

Lors d'une opération 200, la pluralité d'électrodes est reliée à une portion du substrat 3 dont on souhaite connaître les propriétés électriques, et plus exactement la distribution de la densité de charge électrique ou de la conductivité électrique.

Le positionnement des électrodes est illustré sur la [Fig. 3] qui montre une section du substrat 3 à laquelle est reliée la pluralité d'électrodes du dispositif 1. En effet, comme mentionné précédemment, la position de chaque électrode est projetée sur une section du substrat 3 correspondant à l'intersection d'un plan avec la portion du substrat 3.

Dans l'exemple discuté ici, la pluralité d'électrodes comprend dix électrodes respectivement référencées E1, E2, E3, E4, E5, E6, E7, E8, E9 et E10.

Tout d'abord, la section du substrat 3 est divisée en secteurs angulaires définis par un ensemble de droites sécantes en un point de la section du substrat 3.

Dans l'exemple de la [Fig. 3], quatre secteurs angulaires SA1, SA2, SA3 et SA4 sont définis par deux droites D1 et D2 sécantes en un point C de la section du substrat 3. D'une manière générale, un nombre p de droites sécantes en un même point définissent un nombre 2p de secteurs angulaires.

Il doit être noté que le centre C n'est pas nécessairement un centre géométrique de la section du substrat 3. De même, les droites peuvent être quelconques pourvu qu'elles soient sécantes en un même point de la section du substrat 3.

Dans la suite de la description, on appelle « secteurs angulaires opposés » deux secteurs angulaires délimités par les deux mêmes droites. Dit autrement, deux secteurs angulaires opposés sont symétriques par rapport à l'intersection - donc le centre C - des droites. Dans le cas de la [Fig. 3], les secteurs angulaires SA1 et SA3 sont opposés, de même que les secteurs angulaires SA2 et SA4.

Les électrodes sont ensuite reliées à la section du substrat 3 de sorte que deux secteurs angulaires opposés sont tous les deux dépourvus d'électrodes ou tous les deux pourvus d'au moins une électrode. Dit autrement, si au moins une électrode est reliée à un secteur angulaire, alors au moins une électrode est reliée au secteur angulaire opposé ; et si aucune électrode n'est reliée à un secteur angulaire, alors aucune électrode n'est reliée au secteur angulaire opposé.

Dans une variante du motif de mesure discutée plus bas en référence à la [Fig. 4], les électrodes sont reliées à la section du substrat 3 de sorte que deux secteurs angulaires opposés sont tous les deux dépourvus d'électrodes ou tous les deux pourvus d'au moins deux - et non plus une - électrodes.

Le positionnement des électrodes illustré sur la [Fig. 3] est compatible avec cette variante étant donné que les secteurs angulaires opposés SA1 et SA3 sont tous les deux pourvus d'au moins deux électrodes, en l'occurrence trois électrodes E1, E2 et E3 pour le secteur angulaire SA1 et trois électrodes E6, E7 et E8 pour le secteur angulaire SA3, et qu'il en est de même pour les secteurs angulaires opposés SA2 et SA4, en l'occurrence deux électrodes E4 et E5 pour le secteur angulaire SA2 et deux électrodes E9 et E10 pour le secteur angulaire SA4.

Par « électrode reliée à un secteur angulaire », on entend ici que la projection de l'électrode sur la section du substrat 3 est située dans ce secteur angulaire.

Par exemple, le nombre d'électrodes reliées à un secteur angulaire est égal au nombre d'électrodes reliées au secteur angulaire opposé. Ainsi, sur la [Fig. 3], trois électrodes E1, E2 et E3 sont reliées au secteur angulaire SA1 et trois électrodes E6, E7 et E8 sont reliées au secteur angulaire opposé SA3. De même, deux électrodes E4 et E5 sont reliées au secteur angulaire SA2 et deux électrodes E9 et E10 sont reliées au secteur angulaire opposé SA4.

Par ailleurs, les électrodes peuvent être placées sur une ligne fermée L dotée d'un sens de parcours. La ligne fermée L passe par chacun des secteurs angulaires et délimite une zone fermée de la section du substrat 3 comprenant le centre C. La ligne fermée L permet de définir des électrodes voisines qui sont alors deux électrodes consécutives de la ligne fermée L suivant le sens de parcours. Sur la [Fig. 3], le sens de parcours est indiqué par des flèches. À titre d'exemple, les électrodes E3 et E4 sont des électrodes voisines, de même que les électrodes E6 et E7 ou encore que les électrodes E10 et E1.

Il doit être noté que les électrodes ne sont pas nécessairement positionnées à la périphérie de la section du substrat 3.

Comme expliqué précédemment, le substrat 3 peut être liquide ou solide. Dans le cas d'un substrat liquide, les électrodes sont placées sur le contenant. Dans le cas d'un substrat solide, les électrodes sont par exemple directement disposées sur la surface extérieure du substrat 3. Dans le cas d'un matériau multicouche, les électrodes peuvent être disposées sur une des couches ou être en contact avec plusieurs couches.

Les électrodes sont positionnées de manière à délimiter la portion du substrat 3 dont on cherche à déterminer la distribution de la densité de charge électrique ou de la conductivité électrique. Les électrodes sont coplanaires lorsque la portion du substrat 3 est un plan, par exemple la surface extérieure plane d'un matériau ou une couche d'un matériau multicouche. Dans un tel cas, la section du substrat 3 peut coïncider avec la portion du substrat 3. Toutefois, les électrodes peuvent aussi délimiter une portion tridimensionnelle du substrat 3, auquel cas les électrodes ne sont pas coplanaires, d'où l'intérêt de considérer une section du substrat 3 sur laquelle sont projetées les positions des électrodes.

Une fois la pluralité d'électrodes reliée à la portion du substrat 3, l'unité de commande 9 met en œuvre une séquence d'alimentation successive de plusieurs doublets d'électrodes.

Pour ce faire, un motif d'injection, c'est-à-dire la manière dont sont formés les doublets d'électrodes à alimenter, est préalablement défini. Comme mentionné précédemment, le motif d'injection utilisé ici est le motif adjacent. En d'autres termes, un doublet d'électrodes est formé de deux électrodes voisines. Le voisinage d'une électrode comprend typiquement les deux électrodes les plus proches situées de part et d'autre de l'électrode considérée. Comme expliqué précédemment, il est aussi possible d'utiliser la ligne fermée L pour définir le voisinage d'une électrode.

L'unité de commande 9 est configurée avec la séquence d'alimentation, c'est-à-dire la liste ordonnée des doublets d'électrodes à alimenter successivement. Avantageusement, pour un nombre N d'électrodes, il est possible d'alimenter N doublets d'électrodes voisines différents.

Dans l'exemple illustré sur la [Fig. 3], les doublets d'électrodes voisines possibles sont : E1-E2, E2-E3,..., E9-E10 et E10-E1. Il est néanmoins possible de n'alimenter qu'une partie seulement de tous les doublets d'électrodes voisines possibles.

Une variable i correspondant à l'identifiant d'un doublet d'électrodes est initialisée à 1. Cette variable permet d'implémenter, dans le procédé illustré sur la [Fig. 2], une boucle dans laquelle les doublets d'électrodes sont successivement alimentés selon la séquence d'alimentation.

Lors d'une opération 210, l'unité de commande 9 commande l'alimentation, par la source 5, du doublet d'électrodes correspondant à la valeur courante de la variable i.

Dans le cas de la [Fig. 3], le doublet d'électrodes correspondant à la valeur 1 de la variable i est par exemple le doublet d'électrodes formé par les électrodes voisines E1 et E2.

Une fois le doublet d'électrodes alimenté par la source 5, l'unité de commande 9 met en œuvre une séquence de mesure entre les deux électrodes de plusieurs paires d'électrodes.

Pour ce faire, un motif de mesure, c'est-à-dire la manière dont sont formées les paires d'électrodes aux bornes desquelles la tension doit être mesurée, est préalablement défini. Le motif de mesure faisant l'objet de la présente invention a ceci de particulier que chaque paire d'électrodes est formée de deux électrodes de secteurs angulaires opposés.

Le motif de mesure proposé permet de collecter des informations sur le centre de la section du substrat 3 par la mesure de tension - et non par l'injection de courant - entre des électrodes éloignées l'une de l'autre. À l'inverse, le motif opposé et le motif diagonal permettent, certes, d'obtenir des informations sur le centre du substrat, mais par l'injection de courant - la mesure de tension est alors effectuée entre des électrodes voisines -, ce qui implique, comme expliqué précédemment, de créer des directions de propagation du courant opposées.

La [Fig. 4] illustre une variante du motif de mesure qui permet de couvrir plus uniformément la section du substrat 3.

Comme mentionné précédemment, cette variante s'inscrit dans un contexte dans lequel les électrodes sont reliées à la section du substrat 3 de sorte que deux secteurs angulaires opposés sont tous les deux dépourvus d'électrodes ou tous les deux pourvus d'au moins deux électrodes.

Dans cette variante, la condition selon laquelle chaque paire d'électrodes est formée de deux électrodes de secteurs angulaires opposés est assortie d'une condition supplémentaire : deux paires d'électrodes associées à de mêmes secteurs angulaires sont telles que les deux segments reliant chacun, par projection sur la section du substrat 3, les deux électrodes respectives de ces deux paires d'électrodes ne sont pas sécants.

Cette condition supplémentaire est remplie pour tout couple d'une première et d'une deuxième paires d'électrodes dans la mesure où ces première et deuxième paires d'électrodes sont associées aux mêmes secteurs angulaires, c'est-à-dire lorsque les secteurs angulaires opposés auxquels sont respectivement reliées les deux électrodes de la première paire d'électrodes sont les mêmes secteurs angulaires opposés que ceux auxquels sont respectivement reliées les deux électrodes de la deuxième paire d'électrodes.

L'expression « par projection sur la section du substrat 3 » signifie ici que le segment reliant les deux électrodes d'une paire d'électrodes correspond au segment inclus dans la section du substrat 3 et reliant les projections orthogonales respectives de ces deux électrodes sur la section du substrat 3.

Le positionnement des électrodes E1 à E10 sur la [Fig. 4] est le même que sur la [Fig. 3]. Toutefois, la ligne fermée L, les droites D1 et D2, le centre C et les secteurs angulaires SA1, SA2, SA3 et SA4 ne sont pas indiqués sur la [Fig. 4] pour en améliorer la clarté.

Sur la [Fig. 4], les deux électrodes de chaque paire d'électrodes sont reliées par un segment. Comme mentionné plus haut, il apparaît que deux segments reliant chacun, par projection sur la section du substrat 3, les deux électrodes respectives de deux paires d'électrodes de mêmes secteurs angulaires opposés ne sont pas sécants.

En particulier, la paire d'électrodes E1-E8 et la paire d'électrodes E3-E6 sont formées de deux électrodes des mêmes secteurs angulaires, à savoir les secteurs angulaires opposés SA1 et SA3. Les segments reliant respectivement les deux électrodes de la paire d'électrodes E1-E8 et les deux électrodes de la paire d'électrodes E3-E6 ne sont pas sécants.

À l'inverse, la paire d'électrodes E2-E7 et la paire d'électrodes E5-E9 sont formées de deux électrodes de secteurs angulaires distincts, à savoir les secteurs angulaires opposés SA1 et SA3 pour la paire d'électrodes E2-E7 et les secteurs angulaires opposés SA2 et SA4 pour la paire d'électrodes E5-E9. Les segments reliant respectivement les deux électrodes de la paire d'électrodes E2-E7 et les deux électrodes de la paire d'électrodes E5-E9 peuvent être sécants, ce qui est d'ailleurs le cas ici.

Une telle variante du motif de mesure permet, comme illustré sur la [Fig 4], de réaliser un quadrillage de la section du substrat 3, et donc de connaître les propriétés électriques de la section du substrat 3 aussi bien au centre qu'autour de ce dernier.

Au contraire, le motif opposé décrit dans l'article de M. Aller et al. (2022) implique nécessairement que deux segments reliant les deux électrodes respectives de deux paires quelconques d'électrodes soient sécants puisque toute paire d'électrodes est formée de deux électrodes symétriques par rapport au centre du substrat. Ainsi, les propriétés électriques sont bien moins connues à la périphérie du substrat qu'au centre de celui-ci puisque les segments reliant les deux électrodes respectives de toutes les paires d'électrodes sont sécants en un seul et même point : le centre du substrat. D'une manière générale, il n'est pas connu de l'art antérieur d'avoir des segments non sécants entre des secteurs angulaires opposés ; l'article de M. Aller et al. (2022) propose juste de combiner le motif d'injection adjacent avec le motif de mesure opposé sans que de telles considérations soient discutées.

L'unité de commande 9 est configurée avec la séquence de mesure, c'est-à-dire la liste ordonnée des paires d'électrodes aux bornes desquelles la tension doit être mesurée. Typiquement, pour un nombre pair N d'électrodes, il est possible de former N/2 paires d'électrodes de sorte que chaque électrode n'appartient qu'à une seule paire d'électrodes. D'une manière générale, et pour un nombre impair d'électrodes en particulier, une ou plusieurs électrodes peuvent ne pas être sollicitées dans la séquence de mesure.

Une variable j correspondant à l'identifiant d'une paire d'électrodes est initialisée à 1. Cette variable permet d'implémenter, dans le procédé illustré sur la [Fig. 2], une boucle dans laquelle la tension respective entre les deux électrodes de paires d'électrodes est mesurée selon la séquence de mesure.

Lors d'une opération 220, l'unité de commande 9 commande la mesure d'une tension, par le capteur 7, entre les deux électrodes de la paire d'électrodes correspondant à la valeur courante de la variable j.

Dans l'exemple illustré sur la [Fig. 3], la paire d'électrodes correspondant à la valeur 1 de la variable j est par exemple la paire d'électrodes formée par les électrodes E3 et E7. En effet, l'électrode E3 est reliée au secteur angulaire SA1, tandis que l'électrode E7 est reliée au secteur angulaire opposé SA3.

Dans l'exemple illustré sur la [Fig. 4], la paire d'électrodes correspondant à la valeur 1 de la variable j est par exemple la paire d'électrodes formée par les électrodes E4 et E10.

Une fois la mesure de tension réalisée par le capteur 7, la valeur mesurée est transmise au calculateur 11. Cette valeur peut aussi être stockée dans la mémoire 13 de l'unité de commande 9. Alternativement, les valeurs mesurées peuvent n'être transmises au calculateur 11 que lorsque toutes les mesures ont été réalisées.

Lors d'une opération 230, l'unité de commande 9 détermine si le capteur 7 a mesuré la valeur de la tension entre les deux électrodes de toutes les paires d'électrodes de la séquence de mesure.

Si la séquence de mesure n'est pas terminée, la variable j est incrémentée lors d'une opération 240. Les opérations 220 et 230 sont alors répétées jusqu'à ce que la séquence de mesure, prévue en réponse à l'alimentation du doublet d'électrodes correspondant à la valeur courante de la variable i, se termine.

À l'inverse, si la séquence de mesure est terminée, l'unité de commande 9 détermine, lors d'une opération 250, si la source 5 a alimenté tous les doublets d'électrodes de la séquence d'alimentation.

Si la séquence d'alimentation n'est pas terminée, la variable i est incrémentée lors d'une opération 260. Les opérations 210, 220, 230, 240 et 250 sont alors répétées jusqu'à ce que la séquence d'alimentation se termine. En d'autres termes, l'unité de commande 9 commande l'alimentation, par la source 5, d'un nouveau doublet d'électrodes correspondant à la valeur incrémentée de la variable i et une nouvelle séquence de mesure est réalisée.

Il doit être noté que la séquence de mesure peut être différente d'une alimentation d'un doublet d'électrodes à une autre. Par exemple, dans le cas où des secteurs angulaires opposés sont pourvus du même nombre d'électrodes - ce nombre étant nul pour des secteurs angulaires dépourvus d'électrodes -, des secteurs angulaires opposés sont munis d'une bijection entre leurs électrodes respectives pour chaque doublet d'électrodes de la séquence d'alimentation. Ainsi, lorsqu'un doublet d'électrodes de la séquence d'alimentation est alimenté, le motif de mesure est tel que chaque paire d'électrodes est formée de deux électrodes respectivement antécédent et image par la bijection associée aux secteurs angulaires opposés correspondants.

À titre d'exemple, toujours en référence à la [Fig. 3], l'unité de commande 9 peut être configurée de sorte que, lorsque le doublet d'électrodes voisines E6-E7 est alimenté, une bijection soit construite entre les secteurs angulaires opposés SA2 et SA4 et associe les électrodes E4 et E5 respectivement aux électrodes E9 et E10. Par conséquent, la séquence de mesure prévue, pour l'alimentation du doublet d'électrodes voisines E6-E7, consiste à mesurer la tension entre les deux électrodes de la paire d'électrodes E4-E9 et la tension entre les deux électrodes de la paire d'électrodes E5-E10.

Par ailleurs, cette séquence de mesure peut être modifiée pour l'alimentation du doublet d'électrodes voisines suivant E7-E8. En effet, une nouvelle bijection peut être construite entre les secteurs angulaires opposés SA2 et SA4 et associer cette fois-ci les électrodes E4 et E5 respectivement aux électrodes E10 et E9. La séquence de mesure prévue, pour l'alimentation du doublet d'électrodes voisines E7-E8, consiste alors à mesurer la tension entre les deux électrodes de la paire d'électrodes E4-E10 et la tension entre les deux électrodes de la paire d'électrodes E5-E9.

Alternativement, la bijection associée à deux secteurs angulaires opposés est invariante de l'alimentation d'un doublet d'électrodes à une autre.

La séquence de mesure peut aussi être adaptée pour qu'aucune des paires d'électrodes aux bornes desquelles la tension est mesurée ne comprenne l'une des deux électrodes du doublet d'électrodes alimenté.

Il est à nouveau fait référence ci-après à la [Fig. 4]. La variante du motif de mesure illustrée sur cette figure offre moins de liberté pour configurer une séquence de mesure à cause de la condition supplémentaire évoquée plus haut.

Typiquement, une séquence de mesure prévoit que la tension soit mesurée par le capteur 7 entre les deux électrodes de toutes les paires possibles d'électrodes associées selon la variante du motif de mesure. En particulier, lorsque deux secteurs angulaires opposés sont pourvus du même nombre d'électrodes, il existe alors une unique façon de former les paires d'électrodes. À ce titre, il peut être considéré que la [Fig. 4] illustre une séquence de mesure typique dans une configuration dans laquelle chaque secteur angulaire est pourvu du même nombre d'électrodes que le secteur angulaire opposé et dans laquelle la tension est mesurée entre toutes les paires possibles d'électrodes : E1-E8, E2-E7, E3-E6, E4-E10, E5-E9.

Par ailleurs, il est possible là aussi d'adapter la séquence de mesure pour qu'aucune des paires d'électrodes aux bornes desquelles la tension est mesurée ne comprenne l'une des deux électrodes du doublet d'électrodes alimenté.

Dans le cas illustré sur la [Fig. 2], il est prévu d'alimenter successivement les N doublets d'électrodes voisines possibles - soit dix doublets d'électrodes dans l'exemple de la [Fig. 3] - et de mesurer la tension, pour chaque doublet d'électrodes voisines alimenté, entre les deux électrodes de N/2 paires d'électrodes associées selon le motif de mesure - soit cinq paires d'électrodes dans l'exemple de la [Fig. 3]. Avantageusement, les N/2 paires d'électrodes sont formées de sorte que chaque électrode n'appartient qu'à une seule paire d'électrodes. Le nombre de mesures de tension effectuées au total est donc de N²/2 - soit cinquante mesures dans l'exemple de la [Fig. 3].

Comme expliqué précédemment, il est également possible, pour chaque doublet d'électrodes alimenté, d'exclure des mesures les deux électrodes formant le doublet d'électrodes alimenté, ce qui laisse (N-4)/2 paires d'électrodes - soit trois paires d'électrodes dans l'exemple de la [Fig. 3] - formées là aussi de sorte que chaque électrode n'appartient qu'à une seule paire d'électrodes. Le nombre de mesures de tension effectuées au total est alors de N(N-4)/2 - soit trente mesures dans l'exemple de la [Fig. 3].

Les mêmes considérations quant au nombre de mesures s'appliquent à la variante du motif de mesure illustrée sur la [Fig. 4].

Le procédé de TIE proposé, et plus exactement le motif de mesure, permet de réduire significativement le nombre de mesures de tension par rapport à la combinaison du motif opposé et du motif diagonal décrit par B. Loyola et al. (2013) ou au motif en étoile de M. Haingartner et al (2021).

Enfin, lors d'une opération 270, le calculateur 11 cartographie, à partir des mesures de tension, la densité de charge électrique ou la conductivité électrique de la portion du substrat 3. Généralement, la distribution de la densité de charge électrique est plus adaptée pour les substrats liquides, tandis que la distribution de la conductivité électrique est plus adaptée pour les substrats solides.

Dans le domaine de la TIE, il est connu d'avoir recours à la théorie des problèmes inverses pour connaître les propriétés électriques d'un substrat. Le principe de cette approche est de déterminer la cause - ici la distribution de la densité de charge électrique ou de la conductivité électrique - à partir des effets - ici les mesures de tension entre les deux électrodes des paires d'électrodes. En effet, chaque changement dans la distribution de la conductivité électrique entraîne des changements dans la distribution du potentiel et, par conséquent, des changements dans la tension entre les électrodes.

Un problème inverse de la TIE est un problème non linéaire et mal posé. En particulier, ce problème inverse ne dépend pas de façon continue des mesures de tension. Comme rappelé par S.R. Kupis dans la publication « Methods for the Electrical Impedance Tomography Inverse Problem: Deep Learning and Regularization with Wavelets » (All Theses, 2021), il est possible d'utiliser un algorithme de Gauss-Newton de type MIRGN (acronyme anglophone pour « modified iteratively regularized Gauss-Newton ») ou une régularisation de Tikhonov pour résoudre un problème inverse de la TIE.

Par ailleurs, il existe actuellement des solveurs capables de reconstruire la distribution de la densité de charge électrique ou de la conductivité électrique à partir des mesures de tension. Par exemple, le logiciel libre EIDORS fonctionnant sous MATLAB (marque enregistrée) peut être utilisé pour résoudre un problème inverse.

## Revendications

1. Système de tomographie par processus électrique, comprenant un dispositif (1) et un substrat (3),
dans lequel le dispositif (1) comprend :
- une pluralité d'électrodes (E1,...,EN) reliée à une portion du substrat (3),
- une source (5) d'alimentation électrique agencée pour être connectée à un doublet d'électrodes de ladite pluralité d'électrodes (E1,...,EN),
- un capteur (7) de tension agencé pour être connecté à une paire d'électrodes de ladite pluralité d'électrodes (E1,...,EN),
- une unité de commande (9) agencée pour commander l'alimentation successive par la source (5) d'alimentation électrique de doublets d'électrodes formés chacun de deux électrodes voisines, et pour commander, à chaque fois qu'un doublet d'électrodes est alimenté, des mesures de tension respectives par le capteur (7) de tension, chacune entre deux électrodes de paires d'électrodes associées selon un motif de mesure, et
- un calculateur (11) agencé pour cartographier, à partir des mesures de tension, la densité de charge électrique ou la conductivité électrique de la portion du substrat (3),
dans lequel une section du substrat (3), définie par l'intersection d'un plan avec la portion du substrat (3), est divisée en secteurs angulaires (SA1, SA2, SA3, SA4) par un ensemble de droites (D1, D2) sécantes en un même point (C) de ladite section du substrat (3), deux secteurs angulaires délimités par les deux mêmes droites formant des secteurs angulaires opposés,
la pluralité d'électrodes (E1,...,EN) étant reliée à la portion du substrat (3) de sorte que, par projection sur la section du substrat (3), deux secteurs angulaires opposés sont tous les deux dépourvus d'électrodes ou tous les deux pourvus d'au moins deux électrodes,
ledit système étant **caractérisé en ce que** le motif de mesure respecte les deux conditions suivantes :
• chaque paire d'électrodes du motif de mesure est formée de deux électrodes de secteurs angulaires opposés, et
• deux paires d'électrodes du motif de mesure associées à de mêmes secteurs angulaires sont telles que les deux segments reliant chacun, par projection sur la section du substrat (3), les deux électrodes respectives desdites deux paires d'électrodes du motif de mesure ne sont pas sécants.

2. Système selon la revendication 1, **caractérisé en ce que** la pluralité d'électrodes (E1,...,EN) est reliée par projection sensiblement à la périphérie de la section du substrat (3).

3. Système selon la revendication 1 ou 2, **caractérisé en ce que** la pluralité d'électrodes (E1,...,EN) est reliée par projection à une ligne fermée (L) dotée d'un sens de parcours de la section du substrat (3) et **en ce que** deux électrodes voisines sont deux électrodes consécutives de la ligne fermée (L) suivant le sens de parcours.

4. Système selon l'une des revendications précédentes, **caractérisé en ce que** le calculateur (11) est agencé pour cartographier la densité de charge électrique ou la conductivité électrique de la portion du substrat (3) par la résolution d'un problème inverse dont des entrées sont les mesures de tension.

5. Système selon l'une des revendications précédentes, **caractérisé en ce que** la source (5) d'alimentation électrique est une source de tension ou une source de courant.

6. Procédé de tomographie par processus électrique d'un substrat (3), comprenant les opérations suivantes :
- relier (200) une pluralité d'électrodes (E1,...,EN) à une portion du substrat (3),
- alimenter (210) successivement par une source (5) d'alimentation électrique des doublets d'électrodes formés chacun de deux électrodes voisines,
- pour chaque alimentation d'un doublet d'électrodes, effectuer des mesures de tension respectives (220) par un capteur (7) de tension, chacune entre deux électrodes de paires d'électrodes associées selon un motif de mesure, et
- cartographier (270) par un calculateur (11), à partir des mesures de tension, la densité de charge électrique ou la conductivité électrique de la portion du substrat (3),
dans lequel une section du substrat (3), définie par l'intersection d'un plan avec la portion du substrat (3), est divisée en secteurs angulaires (SA1, SA2, SA3, SA4) par un ensemble de droites (D1, D2) sécantes en un même point (C) de ladite section du substrat (3), deux secteurs angulaires délimités par les deux mêmes droites formant des secteurs angulaires opposés,
la pluralité d'électrodes (E1,...,EN) étant reliée à la portion du substrat (3) de sorte que, par projection sur la section du substrat (3), deux secteurs angulaires opposés sont tous les deux dépourvus d'électrodes ou tous les deux pourvus d'au moins deux électrodes,
ledit procédé étant **caractérisé en ce que** le motif de mesure respecte les deux conditions suivantes :
• chaque paire d'électrodes du motif de mesure est formée de deux électrodes de secteurs angulaires opposés, et
• deux paires d'électrodes du motif de mesure associées à de mêmes secteurs angulaires sont telles que les deux segments reliant chacun, par projection sur la section du substrat (3), les deux électrodes respectives desdites deux paires d'électrodes du motif de mesure ne sont pas sécants.

7. Procédé selon la revendication 6, **caractérisé en ce que** la source (5) d'alimentation électrique alimente tous les doublets possibles d'électrodes voisines.

8. Procédé la revendication 6 ou 7, **caractérisé en ce que** la pluralité d'électrodes (E1,...,EN) est reliée à la portion du substrat (3) de sorte que, par projection sur la section du substrat (3), deux secteurs angulaires opposés sont pourvus du même nombre d'électrodes,
**en ce que**, pour chaque alimentation d'un doublet d'électrodes, des secteurs angulaires opposés sont munis d'une bijection entre leurs électrodes respectives, et **en ce que** le motif de mesure est tel que chaque paire d'électrodes est formée de deux électrodes respectivement antécédent et image par la bijection associée aux secteurs angulaires opposés correspondants.

9. Procédé selon la revendication 8, **caractérisé en ce que** la bijection associée à deux secteurs angulaires opposés est invariante de l'alimentation d'un doublet d'électrodes à une autre.

10. Procédé selon la revendication 8 ou 9, **caractérisé en ce que** le capteur (7) de tension effectue des mesures de tension respectives, chacune entre les deux électrodes de toutes les paires possibles d'électrodes associées selon le motif de mesure.

## Patentansprüche

1. System zur elektrischen Prozesstomographie, umfassend eine Vorrichtung (1) und ein Substrat (3),
wobei die Vorrichtung (1) umfasst:
- eine Vielzahl von Elektroden (E1,...,EN), die mit einem Abschnitt des Substrats (3) verbunden ist,
- eine elektrische Stromversorgungsquelle (5), die eingerichtet ist, um mit einem Elektroden-Doublett der Vielzahl von Elektroden (E1,...,EN) verbunden zu werden,
- einen Spannungssensor (7), der eingerichtet ist, um mit einem Elektrodenpaar der Vielzahl von Elektroden (E1,...,EN) verbunden zu werden,
- eine Steuereinheit (9), die eingerichtet ist, um die sukzessive Versorgung von Elektroden-Doubletten, die jeweils aus zwei benachbarten Elektroden gebildet sind, durch die elektrische Stromversorgungsquelle (5) zu steuern, und um jedes Mal, wenn ein Elektroden-Doublett versorgt wird, jeweilige Spannungsmessungen durch den Spannungssensor (7) zu steuern, und zwar jeweils zwischen zwei Elektroden von Elektrodenpaaren, die gemäß einem Messmuster zugeordnet sind, und
- einen Rechner (11), der eingerichtet ist, um aus den Spannungsmessungen die elektrische Ladungsdichte oder die elektrische Leitfähigkeit des Abschnitts des Substrats (3) zu kartieren,
wobei ein Querschnitt des Substrats (3), der durch den Schnitt einer Ebene mit dem Abschnitt des Substrats (3) definiert ist, durch eine Menge von Geraden (D1, D2), die sich in demselben Punkt (C) des Querschnitts des Substrats (3) schneiden, in Winkelsektoren (SA1, SA2, SA3, SA4) unterteilt ist, wobei zwei Winkelsektoren, die durch dieselben zwei Geraden begrenzt sind, entgegengesetzte Winkelsektoren bilden,
wobei die Vielzahl von Elektroden (E1,...,EN) mit dem Abschnitt des Substrats (3) derart verbunden ist, dass durch Projektion auf den Querschnitt des Substrats (3) zwei entgegengesetzte Winkelsektoren beide frei von Elektroden oder beide mit mindestens zwei Elektroden versehen sind,
wobei das System **dadurch gekennzeichnet ist, dass** das Messmuster die folgenden zwei Bedingungen erfüllt:
• jedes Elektrodenpaar des Messmusters aus zwei Elektroden entgegengesetzter Winkelsektoren gebildet ist, und
• zwei Elektrodenpaare des Messmusters, die denselben Winkelsektoren zugeordnet sind, derart sind, dass die zwei Segmente, die jeweils durch Projektion auf den Querschnitt des Substrats (3) die zwei jeweiligen Elektroden der zwei Elektrodenpaare des Messmusters verbinden, sich nicht schneiden.

2. System nach Anspruch 1, **dadurch gekennzeichnet, dass** die Vielzahl von Elektroden (E1,...,EN) durch Projektion im Wesentlichen mit der Peripherie des Querschnitts des Substrats (3) verbunden ist.

3. System nach Anspruch 1 oder 2, **dadurch gekennzeichnet, dass** die Vielzahl von Elektroden (E1,...,EN) durch Projektion mit einer geschlossenen Linie (L) verbunden ist, die mit einer Umlaufrichtung des Querschnitts des Substrats (3) versehen ist, und dass zwei benachbarte Elektroden zwei aufeinanderfolgende Elektroden der geschlossenen Linie (L) entlang der Umlaufrichtung sind.

4. System nach einem der vorhergehenden Ansprüche, **dadurch gekennzeichnet, dass** der Rechner (11) eingerichtet ist, um die elektrische Ladungsdichte oder die elektrische Leitfähigkeit des Abschnitts des Substrats (3) durch Lösen eines inversen Problems zu kartieren, dessen Eingangsgrößen die Spannungsmessungen sind.

5. System nach einem der vorhergehenden Ansprüche, **dadurch gekennzeichnet, dass** die elektrische Stromversorgungsquelle (5) eine Spannungsquelle oder eine Stromquelle ist.

6. Verfahren zur elektrischen Prozesstomographie eines Substrats (3), umfassend die folgenden Schritte:
- Verbinden (200) einer Vielzahl von Elektroden (E1,...,EN) mit einem Abschnitt des Substrats (3),
- sukzessives Versorgen (210) von Elektroden-Doubletten, die jeweils aus zwei benachbarten Elektroden gebildet sind, durch eine elektrische Stromversorgungsquelle (5),
- für jede Versorgung eines Elektroden-Doubletts, Durchführen von jeweiligen Spannungsmessungen (220) durch einen Spannungssensor (7), und zwar jeweils zwischen zwei Elektroden von Elektrodenpaaren, die gemäß einem Messmuster zugeordnet sind, und
- Kartieren (270) der elektrischen Ladungsdichte oder der elektrischen Leitfähigkeit des Abschnitts des Substrats (3) durch einen Rechner (11) aus den Spannungsmessungen,
wobei ein Querschnitt des Substrats (3), der durch den Schnitt einer Ebene mit dem Abschnitt des Substrats (3) definiert ist, durch eine Menge von Geraden (D1, D2), die sich in demselben Punkt (C) des Querschnitts des Substrats (3) schneiden, in Winkelsektoren (SA1, SA2, SA3, SA4) unterteilt ist, wobei zwei Winkelsektoren, die durch dieselben zwei Geraden begrenzt sind, entgegengesetzte Winkelsektoren bilden,
wobei die Vielzahl von Elektroden (E1,...,EN) mit dem Abschnitt des Substrats (3) derart verbunden ist, dass durch Projektion auf den Querschnitt des Substrats (3) zwei entgegengesetzte Winkelsektoren beide frei von Elektroden oder beide mit mindestens zwei Elektroden versehen sind,
wobei das Verfahren **dadurch gekennzeichnet ist, dass** das Messmuster die folgenden zwei Bedingungen erfüllt:
• jedes Elektrodenpaar des Messmusters aus zwei Elektroden entgegengesetzter Winkelsektoren gebildet ist, und
• zwei Elektrodenpaare des Messmusters, die denselben Winkelsektoren zugeordnet sind, derart sind, dass die zwei Segmente, die jeweils durch Projektion auf den Querschnitt des Substrats (3) die zwei jeweiligen Elektroden der zwei Elektrodenpaare des Messmusters verbinden, sich nicht schneiden.

7. Verfahren nach Anspruch 6, **dadurch gekennzeichnet, dass** die elektrische Stromversorgungsquelle (5) alle möglichen Doubletts von benachbarten Elektroden versorgt.

8. Verfahren nach Anspruch 6 oder 7, **dadurch gekennzeichnet, dass** die Vielzahl von Elektroden (E1,...,EN) mit dem Abschnitt des Substrats (3) derart verbunden ist, dass durch Projektion auf den Querschnitt des Substrats (3) zwei entgegengesetzte Winkelsektoren mit der gleichen Anzahl von Elektroden versehen sind, dass für jede Versorgung eines Elektroden-Doubletts entgegengesetzte Winkelsektoren mit einer Bijektion zwischen ihren jeweiligen Elektroden versehen sind, und dass das Messmuster derart ist, dass jedes Elektrodenpaar aus zwei Elektroden gebildet ist, die jeweils ein Urbild und ein Bild durch die den entsprechenden entgegengesetzten Winkelsektoren zugeordnete Bijektion darstellen.

9. Verfahren nach Anspruch 8, **dadurch gekennzeichnet, dass** die zwei entgegengesetzten Winkelsektoren zugeordnete Bijektion von einer Versorgung eines Elektroden-Doubletts zu einer anderen invariant ist.

10. Verfahren nach Anspruch 8 oder 9, **dadurch gekennzeichnet, dass** der Spannungssensor (7) jeweilige Spannungsmessungen durchführt, und zwar jeweils zwischen den zwei Elektroden aller möglichen Elektrodenpaare, die gemäß dem Messmuster zugeordnet sind.

## Claims

1. Electrical process tomography system, comprising a device (1) and a substrate (3),
wherein the device (1) comprises:
- a plurality of electrodes (E1,...,EN) connected to a portion of the substrate (3),
- an electrical power supply source (5) arranged to be connected to an electrode doublet of said plurality of electrodes (E1,...,EN),
- a voltage sensor (7) arranged to be connected to a pair of electrodes of said plurality of electrodes (E1,...,EN),
- a control unit (9) arranged to control the successive supplying, by the electrical power supply source (5), of electrode doublets each formed of two neighboring electrodes, and to control, each time an electrode doublet is supplied, respective voltage measurements by the voltage sensor (7), each between two electrodes of pairs of electrodes associated according to a measurement pattern, and
- a calculator (11) arranged to map, from the voltage measurements, the electrical charge density or the electrical conductivity of the portion of the substrate (3),
wherein a section of the substrate (3), defined by the intersection of a plane with the portion of the substrate (3), is divided into angular sectors (SA1, SA2, SA3, SA4) by a set of lines (D1, D2) intersecting at a same point (C) of said section of the substrate (3), two angular sectors delimited by the same two lines forming opposite angular sectors,
the plurality of electrodes (E1,...,EN) being connected to the portion of the substrate (3) such that, by projection onto the section of the substrate (3), two opposite angular sectors are both devoid of electrodes or both provided with at least two electrodes,
said system being **characterized in that** the measurement pattern satisfies the following two conditions:
• each pair of electrodes of the measurement pattern is formed of two electrodes of opposite angular sectors, and
• two pairs of electrodes of the measurement pattern associated with the same angular sectors are such that the two segments each connecting, by projection onto the section of the substrate (3), the two respective electrodes of said two pairs of electrodes of the measurement pattern do not intersect.

2. System according to claim 1, **characterized in that** the plurality of electrodes (E1,...,EN) is connected by projection substantially to the periphery of the section of the substrate (3).

3. System according to claim 1 or 2, **characterized in that** the plurality of electrodes (E1,...,EN) is connected by projection to a closed line (L) provided with a direction of travel of the section of the substrate (3) and **in that** two neighboring electrodes are two consecutive electrodes of the closed line (L) along the direction of travel.

4. System according to one of the preceding claims, **characterized in that** the calculator (11) is arranged to map the electrical charge density or the electrical conductivity of the portion of the substrate (3) by solving an inverse problem whose inputs are the voltage measurements.

5. System according to one of the preceding claims, **characterized in that** the electrical power supply source (5) is a voltage source or a current source.

6. Method for electrical process tomography of a substrate (3), comprising the following operations:
- connecting (200) a plurality of electrodes (E1,...,EN) to a portion of the substrate (3),
- successively supplying (210), by an electrical power supply source (5), electrode doublets each formed of two neighboring electrodes,
- for each supplying of an electrode doublet, performing respective voltage measurements (220) by a voltage sensor (7), each between two electrodes of pairs of electrodes associated according to a measurement pattern, and
- mapping (270) by a calculator (11), from the voltage measurements, the electrical charge density or the electrical conductivity of the portion of the substrate (3),
wherein a section of the substrate (3), defined by the intersection of a plane with the portion of the substrate (3), is divided into angular sectors (SA1, SA2, SA3, SA4) by a set of lines (D1, D2) intersecting at a same point (C) of said section of the substrate (3), two angular sectors delimited by the same two lines forming opposite angular sectors,
the plurality of electrodes (E1,...,EN) being connected to the portion of the substrate (3) such that, by projection onto the section of the substrate (3), two opposite angular sectors are both devoid of electrodes or both provided with at least two electrodes,
said method being **characterized in that** the measurement pattern satisfies the following two conditions:
• each pair of electrodes of the measurement pattern is formed of two electrodes of opposite angular sectors, and
• two pairs of electrodes of the measurement pattern associated with the same angular sectors are such that the two segments each connecting, by projection onto the section of the substrate (3), the two respective electrodes of said two pairs of electrodes of the measurement pattern do not intersect.

7. Method according to claim 6, **characterized in that** the electrical power supply source (5) supplies all possible doublets of neighboring electrodes.

8. Method according to claim 6 or 7, **characterized in that** the plurality of electrodes (E1,...,EN) is connected to the portion of the substrate (3) such that, by projection onto the section of the substrate (3), two opposite angular sectors are provided with the same number of electrodes,
**in that**, for each supplying of an electrode doublet, opposite angular sectors are provided with a bijection between their respective electrodes,
and **in that** the measurement pattern is such that each pair of electrodes is formed of two electrodes representing respectively a preimage and an image by the bijection associated with the corresponding opposite angular sectors.

9. Method according to claim 8, **characterized in that** the bijection associated with two opposite angular sectors is invariant from one supplying of an electrode doublet to another.

10. Method according to claim 8 or 9, **characterized in that** the voltage sensor (7) performs respective voltage measurements, each between the two electrodes of all possible pairs of electrodes associated according to the measurement pattern.
